# EUROPEAN PATENT APPLICATION

(11) **EP 1 369 477 A1**
(43) Date of publication of application: **10.12.2003**
(21) Application number: 02705113.5
(22) Date of filing: 13.03.2002
(51) Int. Cl.: C12N 15/09, C12Q 1/68

(54) **METHOD OF CONSTRUCTING cDNA TAG FOR IDENTIFYING EXPRESSED GENE AND METHOD OF ANALYZING GENE EXPRESSION**

(30) Priority: 15.03.2001 JP 2001073959
(71) Applicant: Kureha Chemical Industry Co., Ltd., Tokyo 103-0012 (JP); Yamamoto, Mikio, Tokorazawa-shi, Saitama 359-0042 (JP); Yamamoto, Naoki, Tokyo 145-0063 (JP)
(72) Inventor: YAMAMOTO, Mikio, Tokorozawa shi, Saitama 359-0042 (JP); YAMAMOTO, Naoki, Ota-ku, Tokyo 145-0063 (JP); HIROSE, Kunitaka, Nerima ku, Tokyo 179-0074 (JP); KASAI, Jun, Shinjuku ku, Tokyo 169-0073 (JP)
(74) Representative: Curtis, Philip Anthony
(86) International application number: JP0202338
(87) International publication number: WO02074951

(57) **Abstract**

There are provided a method for the preparation of cDNA tags for identifying expressed genes and a method for analysis of gene expression. The cDNA tags for identifying expressed genes are prepared by the method comprising a kind of type II restriction enzyme, two kinds of type IIS restriction enzymes and linkers X and Y having a recognition site for one of two kinds of type IIS restriction enzymes. The cDNA tags can be used alone or in combination like chain (concatemer) formed by combining process to analyze gene expression.

## Description

### (Technical Field)

The present invention relates to a method for the preparation of cDNA tags for identifying expressed genes, a cDNA library prepared by the method and a method for the analysis of gene expression. More specifically, the invention relates to the method for the preparation of cDNA tags hybridizing to mRNAs as products of expressed genes, cDNAs corresponding to the mRNAs or given areas of the cDNA fragments, and the method for analysis of gene expression using the cDNAs. The method for the analysis of gene expression includes a direct method using the cDNA tags without any processing and an indirect method using a concatemer of the cDNA tags.

### (Background Art)

Each species has the peculiar gene expression pattern based on the original genomic sequence. In addition, even if the species is the same, it has been found that each cell or organ shows different gene expression patterns depending on a physiological stage such as degree of differentiation, multiplication and aging, or a pathological state such as canceration, infectious disease and immunologic disease. Accordingly, when such gene expression patterns are compared with each other, the differences provides valuable information which can be used in a variety of applications such as identification of appropriate treatment targets, identification of candidate genes for a gene therapy, tissue typing, legal gene confirmation, positioning of disease related genes and identification of indicator genes for diagnosis.

Northern blotting method, RNase protection method and Reverse transcriptase-polymerase chain reaction (RT-PCR) analysis method (Alwine et al., Proc. Natl. Acad. Sci. U.S.A., 74:5550, 1977; Zinn et al., Cell, 34:865, 1983; Veres et al., Science, 237:415, 1987) were designed in order to evaluate gene expressions. Further methods good for retrieving genes such as expressed sequence tag: EST (Adams et. al, Science 252:1656, 1991; Adams et al., Nature,355: 632, 1992; Okubo et al., Nature Genetics, 2:173, 1992) have been developed. However the methods can evaluate the limited number of genes at a time. For example, Okubo et al. developed a method for obtaining a profile of gene expression comprising the steps of cleaving double-stranded cDNAs with the restriction enzyme having a four base recognition site (Sau3AI) to prepare a cDNA library consisting of 3'-end fragments of the mRNAs, cloning the 3'-end fragments and then sequencing randomly (Okubo et al., Nature Genetics, 2: 173, 1992). Since the method provides the clones having the length of about 300 bases on average and requests sequencing each of the clones separately, a total number of the mRNAs that were finally sequenced in a cell were only about 1,000. As a result, the profile from the method was far from the true pattern of the gene expression. Further, since these methods need lots of samples (for example human tissue), cause bias of the results by repeating the polymerase chain reaction (PCR) and lack reproducibility of the results, they have been used merely in laboratories.

Recently, a method for the serial analysis of gene expression (referred to as SAGE) has been developed (see W097/10363, US applications Nos. 5,695,937 and 5,866,330). The SAGE can analyze lots of expressed genes by identifying a given region of the transcripts corresponding to the expressed genes. In this method, the patterns of gene expression are determined by preparing tags referred to as "ditag" which are formed by ligating randomly two of short nucleotides corresponding to each cDNA in a sample, connecting the ditags like a chain to form concatemers, cloning and sequencing each concatemer to determine the pattern of gene expression. The SAGE cannot provide a single cDNA tag for identifying expressed gene corresponding to each cDNA in a sample and the number of expressed genes to be identified at a time is limited to 1,000 or less, usually 400 or less because the concatemer can contain the limited number of the ditags.

### (Disclosure of Invention)

The present invention provides a method for the preparation of cDNA tags for identifying expressed genes, which enables to conduct the efficient analysis of peculiar gene expression pattern of each species, and of specific gene expression patterns depending on a physiological state, on a development step, or on a pathological states of cells or organs, and also provides a method for the gene expression using the cDNA tags. The method of the present invention requests a less amount of cell samples for the analysis of gene expression and is more efficient and reliable than the conventional technologies. The term "Expressed Gene Identification cDNA tag" as used herein may be abbreviated as EGI cDNA tag or EGI tag, if necessary.

The present invention provides a method for the preparation of cDNA tags for identifying expressed genes comprising: providing complementary deoxyribonucleic acids (cDNAs); cleaving the cDNAs with a type II restriction enzyme to prepare cDNA fragments; ligating the cDNA fragments to linker Xes which have a recognition site of first type IIS restriction enzyme and which form a recognition site of second type IIS restriction enzyme at the site linking with the cleavage end of the cDNA fragments formed by the type II restriction enzyme to prepare linker X-cDNA fragment complex; cleaving the linker X-cDNA fragment complexes with the second type II restriction enzyme to prepare linker X-cDNA tag complexes; ligating linker Ys which have a recognition site of the first type IIS restriction enzyme to the cleavage end of the linker X-cDNA tag complexes formed by the second type IIS restriction enzyme to prepare linker X-cDNA tag-linker Y complexes; amplifying the linker X-cDNA fragment-linker Y complexes; and cleaving the amplified products thus obtained with the first type IIS restriction enzymes to prepare the cDNA tags for identifying expressed genes.

The present invention further provides linker X comprising a recognition site of first type IIS restriction enzyme and which forms a recognition site of second type IIS restriction enzyme at the site linking with the cleavage end of the cDNA fragments formed by the type II restriction enzyme.

The present invention further provides a method for the analysis of gene expression wherein the library of cDNA tags prepared by the method described above is contacted with a detector on which nucleic acids to be detected are immobilized.

The present invention further provides a method for the analysis of gene expression comprising the steps of concatenating the cDNA tags for identifying expressed genes prepared by the method described above to form concatemers and sequencing the concatemers. The method for the analysis includes a method for the qualitative analysis of gene expression wherein the concatemers are sequenced and then each of the cDNA tags are sequenced on the basis of the sequences of the concatemers, and a method for the quantitative analysis of gene expression wherein each of the cDNA tags are sequenced and measured in frequency of occurrences on the basis of the sequences of the concatemers.

The present invention further provides a kit for the preparation of cDNA tags for identifying expressed genes wherein the kit comprises a type II restriction enzyme, a first type IIS restriction enzyme, a second type IIS restriction enzyme, linker Xes which have a recognition site of the first type IIS restriction enzyme and which form a recognition site of the second type IIS restriction enzyme at the site linking with the cleavage end of the cDNA fragments formed by the type II restriction enzyme to prepare linker X-cDNA fragment complexes and linker Ys which have a recognition site of the first type IIS restriction enzyme.

The present invention is based on some fundamental principles (The fundamental principles of the present invention will be explained hereinafter.). First, a short nucleotide sequence isolated from a defined region within a gene transcript has sufficient information to identify the transcript. For example, a sequence of 9 bp may have combinations of the ninth power of four, 262,144 and therefore the sequence can identify the same number of the transcripts. Whereas, estimates suggest that the human genome encodes about 80,000 to 200,000 transcripts (Fields, et al, Nature Genetics, 1:345 1994). Principally, if the tags of 9bp are obtained, all of the transcripts of the human genome can be identified. The size of the tag may be shorter, where a subject of the analysis is a lower eukaryote or prokaryote, because the number of transcripts encoded by the genome is lower. For example, a tag of 6 to 7 bp may be sufficient for distinguishing the transcripts in yeast. The present invention can provide cDNA tags of the same length for identifying expressed genes with a variety of lengths and therefore is useful in the analysis of gene expression patterns.

Second, the present invention can provide extremely reduced bias caused by amplification and/or cloning because the invention allows analyzing a gene expression by once amplification of a single short cDNA tag interposed between upstream and downstream linkers.

Third, a library of the cDNA tags prepared according to the present invention can be used to qualitatively or quantitatively detect the cDNAs corresponding to the cDNA tags for the analysis of gene expression patterns.

Fourth, concatemers with or without spacer sequences of the cDNA tags prepared by the method of the present invention allows serial and efficient analysis of gene expression. If necessary, the concatemers may be cloned by vector and the like. Specifically, since the cDNA tags have independent sequences individually, it is easy to sequence each of the concatemers and to singly isolate the cDNA tags from the concatemers.

It is common between the present invention and the SAGE method described hereinbefore in the first principle that a tag of short nucleotide sequence has sufficient information to identify the transcript. However the SAGE uses a complexized tag referred to as "ditag". The SAGE is different from the present invention in that the SAGE does not prepare and use a single cDNA tag for identifying expressed gene of the present invention, a library thereof and a concatemer of the single cDNA tags.

### (Brief Description of Drawings)

Figure 1 shows steps (1) to (6) of one embodiment of the method for the preparation of cDNA tags for identifying expressed genes according to the invention. The letter "N" in the figures means any one of A, T, G or C.
Figure 2 shows steps (7) to (10) of one embodiment of the method for the preparation of cDNA tags for identifying expressed genes according to the invention.

### (Preferable Modes for Carrying Out the Invention)

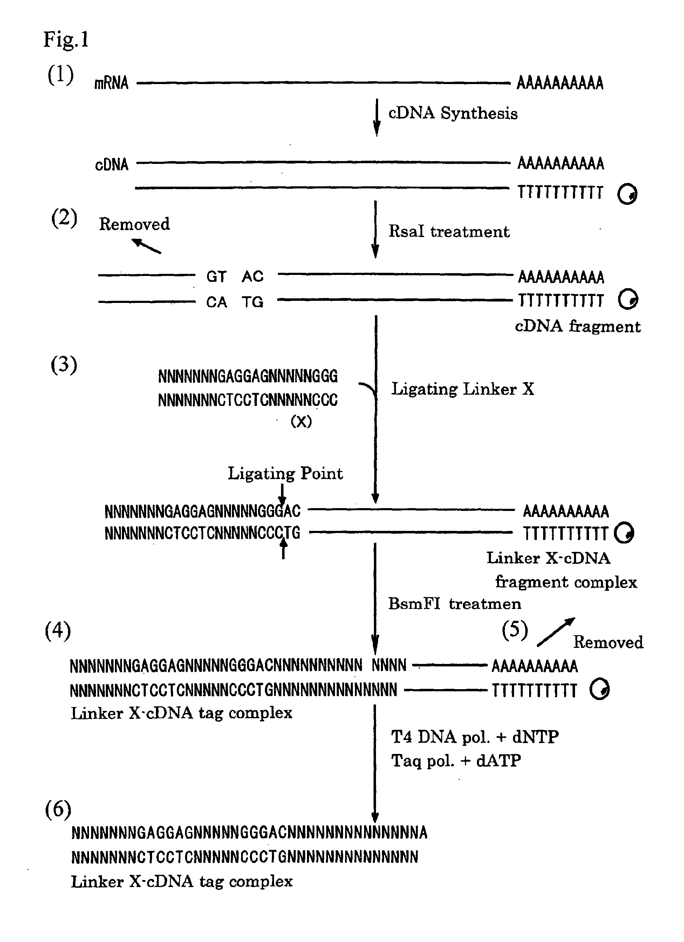

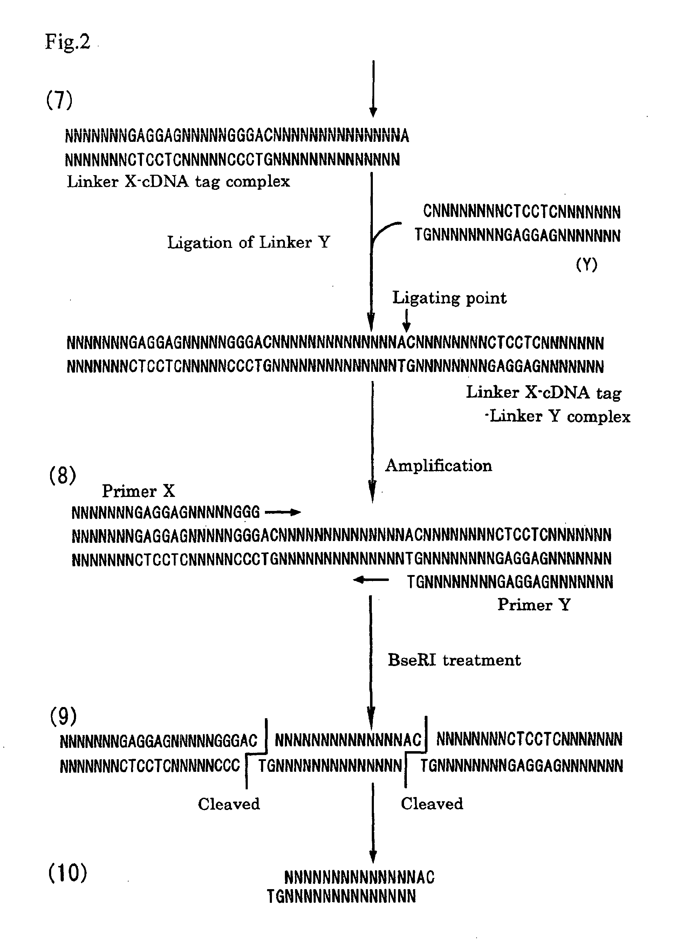

A preferred embodiment of the present invention will be explained in detail using flow-charts shown in figures 1 and 2, of the method for the preparation of cDNA tags for identifying gene expression, which will be referred to as EGI cDNA tag herein after. According to the method, there are easily provided the EGI cDNA tags or a library thereof revealing gene expression of specific cells, tissues or cell extracts in a given developmental stage or a given disease stage.

Figures 1 and 2 show the method of the preparation of cDNA tags for identifying gene expression. The method comprising the steps of:
(1) providing complementary deoxyribonucleic acids (cDNAs);
(2) cleaving the cDNAs with a type II restriction enzyme to prepare cDNA fragments;
(3) ligating the cDNA fragments to linker Xes which have a recognition site of first type IIS restriction enzyme and which form a recognition site of second type IIS restriction enzyme at the site linking with the cleavage end of the cDNA fragments formed by the type 11 restriction enzyme to prepare linker X-cDNA fragment complexes;
(4) cleaving the linker X-cDNA fragment complexes with the second type IIS restriction enzyme to prepare linker X-cDNA tag complexes;
(5) refining the linker X-cDNA tag complexes, if necessary;
(6) processing the cleavage end of the cDNA tags of the linker X-cDNA tag complexes to make the end capable of binding the linker Ys which have a recognition site of the first type IIS restriction enzyme, if necessary;
(7) ligating linker Ys which have the recognition site of the first type IIS restriction enzyme to the cleavage end of linker X-cDNA tag complexes formed by the second type IIS restriction enzyme to prepare linker X-cDNA tag-linker Y complexes;
(8) amplifying the linker X-cDNA fragment-linker Y complexes;
(9) cleaving the amplified products thus obtained with the first type IIS restriction enzyme to prepare the cDNA tags for identifying expressed genes; and
(10) isolating the obtained EGI cDNA tags, if necessary.

In step (1), cDNAs are prepared as a sample. Normally, mRNAs are prepared from the cells to be examined and then the cDNAs are prepared by conventional procedure with a reverse transcriptase. The cDNAs may have sequences corresponding to the full-length mRNAs, fragments of the mRNAs or a combination thereof. The cells to be examined are not limited and may be all the cells including animal cells, plant cells, and microbial cells, where the cells produce mRNAs having a poly A tail at the 3' end. Virus-infected animal cells, plant cells or microbial cells may also be used as the cells to be examined in the invention.

A method of the present invention can analyze a gene expression when one microgram (ug) of mRNAs from the sample is available. Since 1 ug of mRNAs can be normally obtained from 1 mg of the cells to be examined, the present invention is particularly useful in handling precious human tissue samples obtained by needle biopsy.

Isolation of mRNAs from the cells to be examined may be performed by conventional procedure. For example, the mRNAs are obtained by treating the cells with a guanidine reagent or a phenol reagent to isolate total RNAs and then performing an affinity-column method or a batch method using an oligo dT-cellulose or Sepharose 2B as a carrier.

The first chain cDNAs (single-stranded cDNAs) are synthesized with the resultant mRNAs as a template using oligo dT primers and a reverse transcriptase and then the second chain cDNAs (double-stranded cDNAs) are synthesized with the first chain cDNAs as a template. The oligo dT primers used herein include an oligo dT primer immobilized on a solid phase and an oligo dT primer labeled with a coenzyme marker. In light of reproducibility and recovery rate of the targeted cDNA fragments, the oligo dT primer immobilized on a solid phase is preferable. The oligo dT primers immobilized on a solid phase may be oligo dT primers immobilized on latex beads or oligo dT primers immobilized on magnet beads, preferably the oligo dT primers immobilized on magnet beads.

In step (2), the cDNAs in the sample are cleaved with a type II restriction enzyme to prepare the cDNA fragments.

The cDNAs in the sample may be double-stranded cDNAs combined with the oligo dT primers immobilized on a solid phase. The term "type II restriction enzyme" as used herein means a restriction enzyme which recognizes a given recognition site and then cleaves the DNA at a specific position inside of or adjacent to the recognition site. The type II restriction enzyme used in the present invention is a restriction enzyme having at least one recognition site of the mRNA to be analyzed, for example, preferably a type II restriction enzyme having a recognition site consisting of 4, 5 or 6 bases. In particular, taking an average length of the mRNAs, about 2,000 bases into consideration, a type II restriction enzyme having a recognition site of four bases is preferable to the invention because the enzyme theoretically has a recognition site per the fourth power of four, 256 bases.

The type II restriction enzymes used in the invention include Afal, Alul, CviRI, DpnI, HpyCH4V, HpyF44III, Rsal, BfaI, Csp6I, HpyCH4IV, Mael, MaeII, TaqAlphaI, TaqI, TthHB8I, Xspl, Bsp143I, DpnII, MboI, NdeII, Sau3AI, N1aIII, AccII, Bsh1236I, BstUI, BsuRI, FnuDII, HaeIII, MvnI, AciI, BsiSI, HapII, Hin6I, HinP1I, HpaII, MspI, SciNI, CfoI, HhaI, MseI, Tru1I, Tru9I, TasI, Tsp509I and TspEI.

These type II restriction enzymes have a recognition site consisting of all the four bases, A, T, C and G or other recognition site consisting of C and G or A and T.

The type II restriction enzyme having a recognition site consisting of all the four bases, A, T, C and G include Afal, Alul, CviRI, DpnI, HpyCH4V, HpyF44III, RsaI, BfaI, Csp6I, HpyCH4IV, Mael, MaeII, TaqAlphaI, TaqI, TthHB8I, XspI, Bsp143I, DpnII, MboI, NdeII, Sau3AI and N1aIII. The type II restriction enzymes having a recognition site consisting of bases C and G include AccII, Bsh1236I, BstUI, BsuRI, FnuDII, HaeIII, MvnI, AciI, BsiSI, HapII, Hin6I, HinP1I, HpaII, Mspl, SciNI, CfoI and HhaI. In addition, the type II restriction enzymes having a recognition site consisting of bases A and T include MseI, Tru1I, Tru9I, TasI, Tsp509I and TspEI. The restriction enzyme is preferably selected in light of the features of these recognition sites and characteristics of expressed genes to be analyzed.

In this connection, the type II restriction enzyme should be selected so as to make the cleavage end which froms a recognition site of desired second type IIS restriction enzyme at the linking site between the cDNA fragment and the linker X, when the cDNA fragment obtained by step (2) is ligated to the linker X in step (3) as shown in figure 1. For example, where BsmFI having a recognition site of "5'-GGGAC-3"' is selected as a second type IIS restriction enzyme, the linker X with 3'-end of "5'-GGG-3"' and the cDNA fragment with 5'-end of "5'-AC-3"' may be used in order that the linking site between the cDNA fragment and the linker X accords with the recognition site .Accordingly, step (2) may employ type II restriction enzyme such as RsaI and AfaI which has the recognition site of "5'-GTAC -3"' and cleaves the phosphodiester bond between bases "T" and "A".

In step (3), linker Xes which have recognition site of the first type IIS restriction enzyme and which form a recognition site of second type IIS restriction enzyme at the site linking with the cleavage end of the cDNA fragments formed by the type II restriction enzyme are ligated to the cDNA fragment to prepare the linker X-cDNA fragment complexes.

cDNA fragments having oligo dT primer sequences are isolated from the group of the cDNA fragments prepared in step (2). The isolation may be performed by using a label of oligo dT primer. For example, where oligo dT primers immobilized on latex beads are used for the preparation of said cDNAs, the cDNAs may be treated with type II restriction enzyme, centrifuged to precipitate in the form of cDNA fragments having the oligo dT primer sequences immobilized on the beads and then isolated. The cDNA fragments thus obtained are those having a poly A tail and a cleavage-end of said type II restriction enzyme which first appears in the 5' upstream direction from the poly A tail. In the next process, the cDNA fragments are ligated to linker Xes using DNA ligase such as T4 DNA ligase.

The term "linker X" as used herein means a linker which has a recognition site of first type IIS restriction enzyme and which forms a recognition site of second type IIS restriction enzyme at the site linking with the cleavage end of the cDNA fragments formed by the type II restriction enzyme. The recognition site of first type IIS restriction enzyme in linker Xes is preferably located at an appropriate position so that the first type IIS restriction enzyme cleaves the cDNA tag leaving no spacer sequence or a desired spacer sequence.

For example, where linker X has a recognition site of BseRI as that of the first type IIS restriction enzyme and is ligated to the cDNA fragment prepared with Rsal as a type II restriction enzyme, the linker X may be a double-stranded DNA fragment having the following structure.

5'-···GAGGAGNNNNNGGG···-3'          (SEQ ID NO: 1)

3'-···CTCCTCNNNNNCCC···-5'     (SEQ ID NO: 2)

The sequence "5'-GAGGAG-3' "in the linker X is the recognition site of first type IIS restriction enzyme BseRI. The sequence "5'-GGG-3' " at 3' end of the linker X is intended to form recognition site "5'-GGGAC-3' " of BsmFI by ligating with the cleavage end "5'-AC-3' " of the cDNA fragment formed by RsaI. The letter "N" or "n" in base sequence as used herein means any one of bases A, T, C and G.

The term "first type IIS restriction enzyme" as used herein, in principle, means a type IIS restriction enzyme which can recognize commonly recognition sites on linkers X and Y and which forms a desired EGI cDNA tag, or a type I or III restriction enzyme which has the same function as that of the type IIS restriction enzyme.

The first type IIS restriction enzymes used in the invention include MmeI, BpmI, BsgI, BspGI, Eco57I, GsuI, BsmFI, BcefI, FokI, BbvI, Bsp423I, Bst71I, R1eAI, EciI, BseMII, BseRI, HgaI, LweI, SfaNI, AprI, BspMI, HphI, MboII, MnII, BbsI, BciVI, BbvII, BpiI, Bp1I, BpuAI and Faul.

Among these enzymes, the first type IIS restriction enzymes having a distance of ten or more bases from the recognition site to the farthest cleavage end include MmeI, BpmI, BsgI, BspGI, Eco57I, GsuI, BsmFI, BcefI, FokI, BbvI, Bsp423I, Bst71I, R1eAI, EciI, BseMII, BseRI and HgaI. The first type IIS restriction enzymes having the distance of 16 bases or more include MmeI, BpmI, BsgI, BspGI, Eco57I and GsuI.

The term "second type IIS restriction enzyme" as used herein, in principle, means a type IIS restriction enzyme which can recognize a recognition site formed in a linking site of linker X and cDNA fragment on the Linker X-cDNA fragment complex and which cleaves the cDNA fragments at an appropriate point, or a type I or III restriction enzyme which has the same function as that of the type IIS restriction enzyme. The linker X-cDNA tag complex is prepared by cleaving with the second type IIS restriction enzyme.

The second type IIS restriction enzymes include Mmel, Bpml, Bsgl, BspGI, Eco57I, GsuI, BsmFI, BcefI, FokI, Bbvl, Bsp423I, Bst71I, R1eAI, EciI, BseMII, BseRI, HgaI, LweI, SfaNI, AprI, BspMI, HphI, MboII, Mn1I, BbsI, BciVI, BbvII, BpiI, BplI, BpuAI and Faul.

Among these enzymes, the second type IIS restriction enzymes having a distance of ten bases or more from the recognition site to the farthest cleavage end include MmeI, BpmI, BsgI, BspGI, Eco57I, Gsul, BsmFl, Bcefl, Fokl, Bbvl, Bsp423I, Bst71I, R1eAI, EciI, BseMII, BseRI and HgaI. The second type IIS restriction enzymes having the distance of 16 bases or more include MmeI, BpmI, BsgI, BspGI, Eco57I, and GsuI.

Since there is no need to define a sequence of cleavage site formed by the first type IIS restriction enzyme, a combination of the first and second type IIS restriction enzymes is not limited. In contrast, the type II restriction enzyme should be the enzyme which can form a linker X-cDNA fragment complex having a recognition site of the second type IIS restriction enzyme. For example as shown in the table below, there are combinations of the type II restriction enzyme and the second type IIS restriction enzyme.

| Type II | 2nd type IIS | Tag Length |
|---|---|---|
| Afal | MmeI | 20+4bp *2 |
| RsaI | MmeI | 20+4bp *2 |
| AfaI | BsmFI | 14+4bp |
| RsaI | BsmFI | 14+4bp |
| CviRI | R1eAI | 12+4bp *3 |
| HpyCH4V | R1eAI | 12+4bp *3 |
| HpyF44III | R1eAI | 12+4bp *3 |
| Acil | HgaI | 10+4bp |
| Hhal | HgaI | 10+4bp |
| Hin6I | HgaI | 10+4bp |
| SciNI | HgaI | 10+4bp |
| HinP1I | HgaI | 10+4bp |
| DpnI | LweI | 9+4bp |
| Dpnl | SfaNI | 9+4bp |
| DpnI | MnII | 7+4bp *1 |
| Afal | BbsI | 6+4bp |
| RsaI | BbsI | 6+4bp |
| Afal | BbvII | 6+4bp |
| RsaI | BbvII | 6+4bp |
| AfaI | Bpil | 6+4bp |
| RsaI | BpiI | 6+4bp |
| Afal | Bp1I | 6+4bp |
| RsaI | Bp1I | 6+4bp |
| AfaI | BpuAI | 6+4bp |
| RsaI | BpuAI | 6+4bp |
| Acil | Faul | 6+4bp |
| CfoI | FauI | 6+4bp |
| HhaI | FauI | 6+4bp |
| Hin6I | FauI | 6+4bp |
| HinP1I | FauI | 6+4bp |
| SciNI | Faul | 6+4bp |

Denotation (*) at the right side of the table means the combination of type II and second type IIS restriction enzymes which requests a radom sequence in linker Y, since the cleaving site of positive chain is farther than the cleaving site of complementary strand. Figures at the right side of denotation (*) means the number of bases in the random sequence.

In step (4), the linker X-cDNA fragment complexes are cleaved with the second type IIS restriction enzyme to prepare the linker X-cDNA tag complexes. For example, where BsmFI is used as a second type IIS restriction enzyme, the enzyme recognizes the double-stranded DNA containing the recognition site "5'-GGGAC-3' " on the linker X-cDNA fragment complex and the complementary sequence and then cleaves the site "5'-GGGAC-3'(10/14)". That is, BsmFI cuts a phosphodiester bond between the bases located at 10 bp and 11 by 3'- downstream from the base "C" of the 3'-end of recognition site "5'-GGGAC-3' " and a phosphodiester bond between the bases located at 14 bp and 15 bp 5' upstream from the base "G" of 5'-end of complementary chain "3'-CCCTG-5' " of the recognition site "5'-GGGAC-3' ". The resultant DNA fragment has the cleavage end having the following structure.

5'-···GGGACNNNNNNNNNN ···-3' (SEQ ID NO: 3)

3'-···CCCTGNNNNNNNNNNNNNNNN···-5'          (SEQ ID NO: 4)

In step (5), the linker X-cDNA tag complexes obtained in step (4) by cleaving the linker X-cDNA fragment complexes with the second type IIS restriction enzyme are refined, if necessary. This refinement may be done by, as described in step (3), removing the rest of the cDNA fragments cut away from the cDNA tags using oligo dT primers. For example, where the oligo dT primer immobilized on latex beads is used in the preparation of the cDNAs, the precipitation of latex beads is exploited, the solution of the cDNAs treated with the restriction enzyme may be centrifuged to precipitate and then remove the cDNA fragments having labeled oligo dT primers. The supernatant from the centrifugation includes the linker X-cDNA tag complexes.

In step (6), the ends of the cDNA tags in the linker X-cDNA tag complexes are processed to ligate linker Y having recognition site of the first type IIS restriction enzyme.

The methods for processing include a method comprising adding DNA polymerase and dNTP to the solution, the rest of cDNA fragments having labeled oligo dT primers, cut away from the cDNA tag, is removed therefrom, to make the protrusive single stranded end into double stranded blunt end. Further one base "adenine" is added to the 3'-end by adding Taq polymerase and dATP. For example, said cleavage ends formed by processing with type IIS restriction enzyme BsmFI will have the following structure by processing with Taq polymerase. The underlined part of the sequence is newly synthesized.

5'-···GGGACNNNNNNNNNNNNNNNA···-3'          (SEQ ID NO: 5)

3'-···CCCTGNNNNNNNNNNNNNNN ···-5'          (SEQ ID NO: 4)

In step (7), linker Ys are ligated to the cleavage ends of the linker X-cDNA tag complexes formed by the second type IIS restriction enzyme to prepare linker X-cDNA tag-linker Y complexes.

Linker Y is ligated to the linker X-cDNA tag complex with the processed end using a DNA ligase such as T4 DNA ligase. The term "linker Y" as used herein means a linker having a recognition site of first type IIS restriction enzyme, for example BseRI. The recognition site is preferably located so that the type IIS restriction enzyme may cleave the cDNA tags out without leaving spacer sequence(s) or with leaving appropriate spacer sequence(s). For example, the linker Y which ligates the DNA fragment with one additional base of adenine at the 3'-end obtained in step (6) is the DNA fragment having the following structure.

5'-···GAGGAGNNNNNNNNGT···-3'     (SEQ ID NO: 6)

3'-···CTCCTCNNNNNNNNC ···-5'     (SEQ ID NO: 7)

The step provides with the complex having the structure "5'-[linker X]-[cDNA tag (EGI cDNA tag)]-[linker Y]-3"'.

In step (8), the linker X-cDNA tag-linker Y complexes are amplified.

The complexes obtained by step (7) have sequences in linkers X and Y to which primers X and Y may hybridize respectively and may be easily amplified by polymerase chain reaction (PCR). The standard polymerase chain reaction method may be used for the present invention, for example the method described in USP No. 4,683,195. Further the complex may be amplified by cloning the one that is ligated into a vector adaptable to a prokaryote or by another method for the amplification known to those skilled in the art.

Where the PCR is performed using template mixtures comprising a variety of DNAs having a different length which are ligated to the linkers for primer annealing at ends thereof, the amplification efficiency varies depending on the length of each template DNA. Generally, as the strands are long, the efficiency of the amplification becomes lower. As the strands shorten, the efficiency becomes higher. As a result, an occurrence ratio of each amplified fragment in the amplified products thus obtained does not reflect the abundance ratio of corresponding the DNA fragment in the mixture of the template DNA. In contrast, since the template DNA used in present invention have the same length and is short, the occurrence ratio of each amplified DNA fragment in the resultant amplified products should reflect the abundance ratio of the corresponding DNA fragment in the mixture of template DNAs. Because, theoretically, there is hardly influence due to the difference in amplification efficiency of PCR, the occurrence ratio of each amplified DNA fragment in the resultant amplified products will reflect the ratio of the corresponding mRNA in the mRNAs expressed in the cells to be examined.

The PCR method may be performed under standard conditions of time and temperature in the present invention. Since the linker X-cDNA tag-linker Y complex used in the invention provides a high efficiency of amplification due to its short and equal in length, the number of annealing/sequence extension cycles may be reduced. In addition, since an efficiency of the PCR method may vary due to a change in the sequence of linker, appropriate linkers used in the procedure may give a desired efficiency of annealing/sequence extension cycle.

The term "primer X" as used herein means a naturally-occurring or synthesized oligo nucleotide which is complementary to a nucleic acid strand of linker X and may work as an initiation point under conditions that the PCR starts. The primer X should have a length enough to hybridize at the site where a recognition site of the first type IIS restriction enzyme on linker X is retained and to initiate the amplification in the presence of an agent for polymerization. A required length of primer X will be determined due to lots of factors such as temperature, pH and ligase used in the PCR. Likewise, the term "primer Y" as used herein means a naturally-occurring or synthesized oligo nucleotide which is complementary to a nucleic acid strand of linker Y and may work as an initiation point under conditions that the PCR starts.

Those of skill in the art will easily prepare primers for amplification based on the nucleotide sequence of the linkers by taking the first type IIS restriction enzymes into consideration without undue experimentation.

In step (9), the resultant amplified-products are cleaved with the first type IIS restriction enzymes to produce the cDNA tags for identifying expressed genes. For example, where BseRI is used as a first type IIS restriction enzyme, the enzyme recognizes the double-stranded DNA consisting of sequence "5'-GAGGAG-3"' on the linker X and its complementary strand and then cleaves the site "5'-GAGGAG-3'(10/8)". Namely, BseRI cuts a phosphodiester bond between the bases located at 10 bp and 11 bp 3'- downstream from the base "G" of the 3'-end of recognition site "5'-GAGGAG-3"' and a phosphodiester bond between the bases located at 8 bp and 9 bp 5'-upstream from the base "C" of 5'-end of complementary chain "3'-CTCCTC-5"' of the recognition site "5'-GAGGAG-3' ". The resultant DNA fragment of linker X with the cleavage end having the following structure is prepared.

5'-···GAGGAGNNNNNNNNNN···-3'     (SEQ ID NO: 8)

3'-···CTCCTCNNNNNNNN ···-5' (SEQ ID NO: 9)

Likewise, the first type IIS restriction enzyme, BseRI recognizes the double-stranded DNA consisting of sequence "5'-GAGGAG-3"' on the linker Y and its complementary strand and then cleaves the site "5'-GAGGAG-3'(10/8)". Namely, BseRI cuts a phosphodiester bond between the bases located at 10 bp and 11 bp 3'-downstream from the base "G" of the 3'-end of recognition site "5'-GAGGAG-3"' and a phosphodiester bond between the bases located at 8 bp and 9 bp 5'-upstream from the base "C" of 5'-end of complementary chain "5'-CTCCTC-3"' of the recognition site "5'-GAGGAG-3"'. The resultant DNA fragment of linker Y with the cleavage end having the following structure is prepared. As a result, the EGI cDNA tag is cut out from the DNA fragments including linkers X and Y.

In short, there is provided the EGI cDNA tag consisting of sequences Nos.10 and 11 indicated below, comprising a nucleotide chain of fourteen bases adjacent to Rsal cleavage end (5'-AC-3') of the cDNA fragment derived from the cDNA to be examined, by using RseI as a type II restriction enzyme in step (2), linker X comprising a nucleotide chain having base sequence of SEQ ID NO: 1, BsmFI as a second type IIS restriction enzyme in step (4), linker Y comprising a nucleotide chain having base sequence of SEQ ID NO: 6, and BseRI as a first type IIS restriction enzyme in step (9).

5'-···   NNNNNNNNNNNNNNNAC···-3'          (SEQ ID NO:10)

3'-···TGNNNNNNNNNNNNNN ···-5' (SEQ ID NO:11)

Where the method of the present invention is carried out with a cDNA library obtained from mRNAs derived from cells, a library of the EGI cDNA tags is obtained in step (9).

According to the present invention, the cDNAs corresponding to the EGI cDNA tags for identifying expressed genes can be qualitatively or quantitatively detected to analyze a pattern of gene expression by utilizing the resultant library of the EGI cDNA tags.

For example, a selection of target genes can be conducted by providing a detector having spots of a library of EGI cDNA tags corresponding to cDNAs to be detected, contacting each of a sample obtained from a subject and a standard sample, which are labeled with different markers respectively, with the detector and comparing relative signal strength of the different markers. A wide range of known markers such as fluorescent marker and radio isotopic marker may be used in this step.

According to another aspect of the present invention, cDNA tags in a library of the EGI cDNA tags can be detected to analyze a pattern of the gene expression by contacting the library with a detector to which the cDNAs to be detected are immobilized.

The detectors used for the present invention includes a microarray device such as DNA chip and a macroarray device such as dot hybridization. Substrates used for the detector include Nylon membrane, nitrocellulose filter, glass plate and silicon chip. The detector may be, for example a device for detecting target nucleic acids in which the resultant cDNA tags are immobilized on a substrate and then DNA, RNA and/or their fragments to be detected are hybridized thereon.

Samples are preferably labeled in a manner such that mRNAs or cDNAs can be detected. For example, markers in this step include radioisotope, fluorescent compound, bioluminescence compound, chemiluminescence compound, metal chelator or enzyme.

For example, labeled cDNAs to be detected are melted into single strands, if necessary, gradually diluted and then contacted with a solid substrate holding the cDNA tags corresponding to genes to be detected in each grid of silicone chip. Conditions of cell sample can be easily found by comparing the resultant pattern of gene expression with a standard pattern of gene expression. In addition, an expression pattern of unknown gene can be recorded by fixing cDNA tags of the gene. As a result, the gene will be able to be reanalyzed in future, where the gene is identified.

In the present invention, the length of cDNA tag for EGI may be adjusted by selecting an appropriate combination of second type II restriction enzyme and second type IIS restriction enzyme. Although a desired length of cDNA tag may vary depending on a kind of species to be analyzed, the length of the DNA tag generally ranges from 6 to 25 bp, preferably from 10 to 25 bp and more preferably 10 to 16 bp.

In step (10), the cDNA tag may be isolated, if necessary. The isolation may be conducted by conventional methods used by those skilled in the art such as polyacrylamide gel electrophoresis.

In addition, expressed genes may be determined by ligating the cDNA tags to form a concatemer and then sequencing the concatemer. For example, since the cDNA tags obtained in step (9) have 3'- and 5'-cohesive ends which are complementary to each other, they can be ligated each other with T4 ligase. The resultant concatemer of cDNA tags may be analyzed by methods, known to those skilled in the art, for example, cloning into a vector or sequencing with a sequencer.

In the present invention, concatemers generally consist of 3 to 200 of EGI cDNA tags, preferably from 3 to 80 of EGI cDNA tags and more preferably from 16 to 40 of EGI cDNA tags. In this connection, the resultant concatemer may or may not have a spacer sequence between EGI cDNAs tags depending on methods for the preparation of EGI cDNA tags.

The concatemers of EGI cDNA tag in the present invention may be cloned by standard methods comprising the steps of integrating the tags into plasmids or phages and amplifying.

The term "recombinant vector" as used herein refers to a plasmid, virus or other vehicle prepared by inserting or cloning the concatemer of EGI cDNA tags into it. Such a vector includes an origin of replication, a promoter and a specific gene which allows a phenotypic selection of transformed cell. In the present invention, many kinds of known cloning vectors suitable for sequencing may be used. Examples of such vectors include, pUC18, altered vectors of pUC18 such as pUC118, pUC19, altered vectors of pUC19 such as pUC119, M 13 m p 18RFI, M13mp19RFI, pBR322, pCR3.1, pBAD-TOPO, altered vector of pBAD-TOPO and pBluescript(R)II.

The recombinant vectors are transfected into an appropriate host cell. The term "host cell" as used herein means a cell in which a vector may be amplified and a DNA of the vector may be expressed, or progenies thereof. Since a mutation may occur during their replication, all of the progenies are not always the same as their parent cell.

The present invention may utilize known and stable methods for transferring an exogenous gene by which the gene is continuously retained. For example, where prokaryotic cells such as Escherichia coli are used as a host cell, the cells are harvested after the exponential growth phase and treated by known methods such as RbCI method and CaCl₂ method to prepare competent cells having an ability to uptake DNA. The cells may be transformed by electroporation or conventional methods.

According to the present invention, 20 or more EGI cDNA tags, preferably 20 to 100 EGI cDNA tags and more preferably 20 to 30 EGI cDNA tags can be sequenced in an operation by cloning a concatemer of the EGI cDNA tags into a vector and sequencing the concatemer.

Although the preferred embodiments of the present invention have been described herein before, it will be apparent that those skilled in the art may make a variety of changes and modifications without departing from the scope of the present invention. The present invention will be particularly explained on the basis of the following examples, which are not intended to limit a protective scope of the invention. Namely, it should be understood that the present invention will be limited only by the claims attached to the present application.

### (Examples)

### (Example 1) Analysis of gene expression of peripheral blood lymphocytes

Peripheral blood mononuclear cells (PBMC) were collected from peripheral blood obtained from normal donor with NycoPrep1.077A (Nyco Med Pharma AS). The resultant peripheral blood lymphocytes were incubated at 37 degrees Celsius for three hours in the presence or absence of 10 ug/ml lipopolysaccharide (LPS), and then an total RNA was extracted from the incubated cells using Isogen (Nippon Gene Co. Ltd.). The total RNA extract obtained was treated at 37 degrees Celsius for 30 minutes with DNaseI (Takara Shuzo Co., LTD) and then refined with RNeasy (QIAGEN). The mRNA was isolated from the total RNA by adsorbing with Oligotex-MAG mRNA refinement kit (Takara Shuzo Co., LTD) and then double-stranded cDNAs were prepared from the mRNA by using cDNA synthesis kit (Takara Shuzo Co., LTD).

The resultant double-stranded cDNAs were cleaved by treating with restriction enzyme RsaI (New England Biolabs Inc.) at 37 degrees Celsius for two hours. The cleaved fragments with magnet beads were collected on a wall surface to obtain a fraction including sequences located between a poly A tail of said RNA and the recognition site of RsaI first appeared in the 5'-upstream direction of the poly A tail by using a magnet. Linker X having a recognition site of a first type IIS restriction enzyme BseRI was ligated to the fraction of the cDNA fragments by one of the three processes described below with T4 DNA ligase.
(1) Process for directly ligating linker X to cleavage end of RsaI:
   Linker X having the following structure was directly ligated to the blunt end formed by cleaving with RsaI. 5'-···TGCAGCTGAGGAGTCCATGGG···-3'          (SEQ ID NO:12) 3'-···ACGTCGACTCCTCAGGTACCC···-5'          (SEQ ID NO:13)
(2) Process for ligating linker X to make the blunt cleavage end of Rsal into cohesive by one base addition.
   One base "C" was added to the 3'-blunt end which was formed by cleaving with RsaI, in the following manner (underlined) by processing with Taq DNA polmerace in the presence of dCTP.
   5'-^{...} AC^{...}-3'
   3'-^{...}CTG^{...}-5'
   In the next step, the linker X having the following structure was ligated to said cohesive end. 5'-···TGCAGCTGAGGAGTCCATGGG···-3'     (SEQ ID NO:12) 3'-···ACGTCGACTCCTCAGGTACC ···-5'     (SEQ ID NO:14)
(3) Process for ligating linker X to make the blunt cleavage end of RsaI into cohesive by one base deletion.
   One base "T" was deleted from the 3'-blunt end which was formed by cleaving with RsaI in the following manner (underlined) by processing with T4 DNA polymerase in the presence of dATP, dGTP and dCTP. 5'-^{...}AC^{...}-3'
   3'-^{···}_G^{···}-5'

In the next step, the linker X having the following structure was ligated to said cohesive end by processing at 16 degree Celsius for two hours using T4 DNA ligase.

5'-···TGCAGCTGAGGAGTCCATGGG ···-3'          (SEQ ID NO:12)

3'-···ACGTCGACTCCTCAGGTACCCT···-5'          (SEQ ID NO:15)

The linker X-cDNA fragments were cleaved with BsmFI (New England Biolabs) at 65 degrees Celsius for two hours utilizing the recognition site of restriction enzyme BsmFI "5'-GGGAC-3' " formed by ligating the linker X. The cleaved fragments having no beads, that is, the supernatant was collected. Since the cleavage site of the enzyme was at position 5'-GGGAC-3'(10/14), the collected fragments includes 14 base pairs derived from the cDNA following the linker X (except for common two residues "AC" from RsaI cleavage site).

The supernatant was treated with T4 DNA polymelase at 16 degree Celsius for two hours in the presence of dATP, dCTP, dGTP and dTTP. The fragments were collected and then treated with Z-Taq (Takara Shuzo Co. Ltd) at 70 degree Celsius for 30 minutes in the presence of dATP. By said treatment, a protrusion of base "A" at the 3'-end was occurred. The second linker Y having the following structure was ligated using T4 DNA ligase at 16 degree Celsius for two hours.

5'-··· CATGTGTCGCTCCTCACTAGAC···-3'          (SEQ ID NO:16)

3'-···TGTACACAGCGAGGAGTGATCTG···-5'    (SEQ ID NO:17)

By ligating said linker Ys, there was provided a library of complexes consisting of small cDNA fragments "linker X-AC-14 bp derived from cDNA (EGI cDNA tag)-AC-linker Y", that is, a group of DNAs of whole length 60 bp including 14 bp derived from cDNAs interposed between known linkers. This fragment consists of the base sequence indicated below and its complementary chain.

5'-
···TGCAGCTGAGGAGTCCATGGGACNNNNNNNNNNNNNNACATGTGTCGCTCC
TCACTAGAC···-3'
(SEQ ID NO:18)

The library of complexes consisting of small cDNA fragments were amplified by PCR method using Taq DNA polymerase, primer X comprising base sequence "5'-TGCAGCTGAGGAGTCCATGGG-3"' (SEQ ID NO:12) which hybridizes the linker X region and primer Y comprising base sequence "5'-GTCTAGTGAGGAGCGACACATGT-3' " (SEQ ID NO:17) which hybridizes the linker Y region. The PCR method was performed by melting at 96 degrees Celsius for 30 seconds, annealing at 50 degrees Celsius for one minute and extending at 72 degrees Celsius for one minutes for 25 cycles and then finally extending at 72 degrees Celsius for two minutes.

The obtained PCR products were treated with the type IIS restriction enzyme BseRI (New England Biolabs). Since the recognition sites of the enzyme is "5'-GAGGAG-3'(10/8)", the DNA fragments having the following structure occurred.

5'-···   NNNNNNNNNNNNNNAC···-3'          (SEQ ID NO:10)

3'-···TGNNNNNNNNNNNNNN ···-5' (SEQ ID NO:11)

The treated products were electrophoresed through 12% polyacrylamide gel and isolated from the linker fragments to collect the small fragment DNAs.

After the resultant cDNA tags were ligated each other with T4 ligase to obtain concatemers, they were electrophoresed through 4.5% polyacrylamide gel to collect the concatemers having 500 bp to 1000 bp in length. The collected concatemers have the structure indicated below. Since 5'-AC-3' adjacent to (N)14 was the sequence derived from RsaI recognition site of the cDNA, there was provided a library of concatemers of cDNA tags which were completely derived from the cDNAs and which include no spacer sequences artificially added. In the base sequences below," (N)14 "refers to fourteen bases "5'-NNNNNNNNNNNNNN-3"' (SEQ ID NO:19) derived from the cDNA.

5'-···AC(N)14AC(N)14AC(N)14AC(N)14AC(N)14AC(N)14AC···-3'
(SEQ ID NO:20)

3'-···TG(N)14TG(N)14TG(N)14TG(N)14TG(N)14TG(N)14TG···-5'
(SEQ ID NO:21)

The above concatemer was cloned into plasmid pUC118 and sequenced the base sequence using DNA sequencer (ABI377). As a result, the genes specifically expressed in PBMC or the ones stimulated with LPS were analyzed. It is considered that about 10,000 of tag sequences need to be sequenced in order to approximately identify kinds and estimate appearance ratio of each mRNAs expressed in a cell. Since about 20 EGI cDNA tags could be sequenced in one sequencing operation according to the invention, the kind and the ratio of each mRNA expressed in the specimen can be estimated by determining base sequences of about 500 samples.

Tables 1 and 2 show some genes identified by this method. A homology screening for base sequences of these EGI cDNA tags was carried out by using known database. Table 1 shows the genes which were enhanced by LPS stimulation. Table 2 shows the genes which were suppressed by LPS stimulation.

**Table 1**

| Genes whose expressions are enhanced by LPS stimulation | | |
|---|---|---|
| mf ID | mf Base sequence | Name of Gene |
| 44924901 | 5'-AGGGTCCTTTTGCA-3' (SEQ ID No.22) | hII3.3B Gene for Histon H3.3 (Hs.180877) |
| 261849128 | 5'-TTGCGTGAAAAGCT-3' (SEQ ID No.23) | Arg-Serpin (plasminogen activator-inhibitor 2, PAI-2) (Hs.75716) |
| 88602527 | 5'-CCCACTTTCTGCTG-3' (SEQ ID No.24) | Unknown |
| 220597775 | 5'-TCAGCGAATGAATG-3' (SEQ ID No.25) | IL-1 receptor antagonist, IL-1ra (IL-1RN) Gene, complete codes (IIs.81134) |
| 69402230 | 5'-CAAGAGTTTGCTCC-3' (SEQ ID No.26) | CC chemokine LARC precursor |
| 232235060 | 5'-TCTCCTGGAAATAT-3' (SEQ ID No.27) | Cytokine subfamily B (Cys-X-Cys), Member 10 (SCYA10) mRNA |
| 110001478 | 5'-CGGATGCTTCCACC-3' (SEQ ID No.28) | Interferon Repression factorl (IRF-1) mRNA |
| 247718478 | 5'-TGTAATTGAGCATC-3' (SEQ ID No.29) | (Putative) Initiation factor (SUI-1) mRNA |
| 196314601 | 5'-GTGTATGACCTGGA-3' (SEQ ID No.30) | Activation (Act-2) mRNA complete codes (Hs.75703) |
| 97872251 | 5'-CCTCCCCGGCCTGG-3' (SEQ ID No.31) | JAK Binding protein (SSI-1) mRNA |

**Table 2**

| Genes whose expressions are suppressed by LPS stimulation | | |
|---|---|---|
| mf ID | mf Base sequence | Name of Gene |
| 123160542 | 5'-CTCCCTCACTTCTC-3' (SEQ ID No.32) | Gardner-Rasheed feline sarcoma viral (v-fgr) oncogene homolog (FGR) mRNA |
| 129504303 | 5'-CTGTGAACCAAGTG-3' (SEQ ID No.33) | Liposome protein L3 (RPL3) mRNA |
| 90708255 | 5'-CCCGGAACGCACTG-3' (SEQ ID No.34) | Major histocompatibility complex class II DMα (HLA-DMA) mRNA |
| 70355926 | 5'-CAATACGAGTTCCC-3' (SEQ ID No.35) | Actin-related protein 2/3 complex subunit 1B (41Kd)(ARPC1B) mRNA |
| 233301643 | 5'-TCTGCTTGCGGAGG-3' (SEQ ID No.36) | Homo sapiens zyxin (ZYX) mRNA |
| 90143380 | 5'-CCCCTTCTGGGCAT-3' (SEQ ID No.37) | G(i) Protein α - subunit (Adenylate cyclase inhibiting GTP- binding protein) (Hs.77269) mRNA |
| 77904298 | 5'-CAGGCAGTGCGGGC-3' (SEQ ID No.38) | Apoptosis-associated speck-like protein containing a CARD (ASC) mRNA |
| 208646773 | 5'-TACGTTGTAGCTCA-3' (SEQ ID No.39) | Mitochondrial DNA Complete sequence |
| 68307279 | 5'-CAACAGCAGCCATG-3' (SEQ ID No.40) | Hematogenesis cell protain-tyrosine kinase (HCK) Gene, Complete sequence Lambda-a2 clone (Hs.89555) |
| 237072942 | 5'-TGAGACCTAGAGTC-3' (SEQ ID No.41) | ADP/ATP Translocase mRNA, 3' UTR |

Numbers designated as mf ID before base sequences in tables 1 and 2 are decimal numbers for computer processing which refer to sequences of 14 bases. Namely, the mf ID is a decimal number generated by substituting 0 for a, 1 for c, 2 for g and 3 for t to make a quaternary digit, converting the number to a decimal number and adding one. Base sequences can be processed as a number regardless of their length. For example, when base sequences consisting of 14 bases are processed, the sequences can be identified by using the numbers in a manner indicated below.

5'-···aaaaaaaaaaaaa···-3' (SEQ ID NO:42)   00000001 (or simply referred to as 1)

5'-···aaaaaaaaaaaac···-3' (SEQ ID NO:43)   00000002 (or simply referred to as 2)

5'-···aaaaaaaaaaaaag···-3' (SEQ ID NO:44)    00000003 (or simply referred to as 3)

5'-···aaaaaaaaaaaat···-3' (SEQ ID NO:45)    00000004 (or simply referred to as 4)

5'-···aaaaaaaaaaaaca···-3' (SEQ ID NO:46)   00000005 (or simply referred to as 5)

. . . . . . . . . . . . . . . . . . .

. . . . . . . . . . . . . . . . . . .

5'-···ttttttttttttgt···-3'    (SEQ ID NO:47)    268435452

5'-···ttttttttttttta···-3'    (SEQ ID NO:48)    268435453

5'-···tttttttttttttc···-3'    (SEQ ID NO:49)    268435454

5'-···ttttttttttttg···-3'    (SEQ ID NO:50)    268435455

5'-···tttttttttttttt···-3'     (SEQ ID NO:51)     268435456

By using such IDs, any sequences consisting of 14 bases may be designated one ID number of nine digits. These figures are referred to as mini fragment ID (mf ID).

### (Example 2)

The library of the EGI cDNA tags prepared in example 1 is detected with the detector described below to analyze the gene expression.

A DNA chip is produced by synthesizing oligo DNAs comprising sequences corresponding to mf base sequences designated as mfID261849128, 220597775, 69402230, 232235060, 110001478 and 196314601 of the genes listed in Table 1 whose expression is activated by LPS stimulation, and spotting on a slide glass with the oligo DNAs using a conventional method.

In order to prepare the probe solutions, mRNAs derived from the peripheral blood mononuclear cells (PBMC) obtained by LPS stimulation in example 1 which are used as a template were labeled with a fluorescent marker, fluorescent compound Cy3-dUTP (*1) (Amersham Pharmacia), and other mRNAs derived from PBMC not stimulated with LPS which are used as a template are labeled with a fluorescent marker, fluorescent compound Cy5-dUTP (*5) (Amersham Pharmacia).

The probe solutions are mixed together to make 6 × SET [0.9M NaCI, 10 µ g/ml Yeast tRNA, 0.1%SDS, 120mM Tris-HCl(pH7.8)] solution and then kept in contact with said oligo DNA chip at 45 degrees Celsius overnight to perform hybridization.

After the DNA chip is washed with a washing liquid [6 × SSC, 0.1 %SDS]at 52 degrees Celsius, the fluorescent markers on the chip are scanned with a scanner to obtain the fluorescence intensity data and then the data is analyzed. The scatter plot of signal intensity of Cy3 and Cy5 at each of the spots demonstrated that the fluorescent light radiated by the probes derived from the mRNAs from the PBMC stimulated with LPS is more than twice stronger than that from the PBMC not stimulated with LPS at all of the spots.
*1) CAS RN Cy3 CAS RN 146368-16-3
CN 3H-Indolium, 2-[3-[1-[6-[(2,5-dioxo-1-pyrrolidinyl)oxy]-6-oxohexyl]-1,3-dihydro-3,3-dimethyl-5-sulfo-2H-indol-2-ylidene]-1-propenyl]-1-ethyl-3,3-dimethyl-5-sulfo-, inner salt (9CI) (CA INDEX NAME)
*2) CAS RN Cy5 CAS RN 146368-14-1
CN 3H-Indolium, 2-[5-[1-[6-[(2,5-dioxo-1-pyrrolidinyl)oxy] -6- oxohexyl]-1,3-dihydro-3,3-dimethyl-5-sulfo-2H-indol-2-ylidene]-1,3-pentadienyl]-1-ethyl-3,3-dimethyl-5-sulfo-, inner salt (9CI) (CA INDEX NAME)

### (Example 3)

By using the cDNA tags optionally selected from the library of the EGI cDNA tags obtained in example 1, gene expression differences between a pair of samples can be analyzed.

cDNAs prepared with a reverse transcriptase by using mRNAs derived from peripheral blood mononuclear cells (PBMC) stimulated with LPS and mRNAs derived from PBMC not stimulated with LPS as a template, are spotted on a nylon membrane and then the cDNAs on the membrane are incubated at 80 degrees Celsius for two hours.

An oligo DNA comprising the sequence of mfID261849128 selected from the genes whose expression were induced by LPS stimulation as shown in Table 1 is synthesized and then labeled with [ gamma-³² P ] ATP (Amersham Pharmacia) by T4 polynucleotide kinase to obtain a probe solution including the probe labeled with ³²P (radioisotope).

This probe solution is used to perform a hybridization with said nylon membrane in 6 x SET overnight at 45 degrees Celsius. After the nylon membrane was washed with washing solution [6 x SSC, 0.1% SDS] at 52 degrees Celsius, and then performed autoradiography. The signals on X-ray film of the cDNA derived from the mRNA of LPS stimulated PBMC was twice stronger than those of LPS non-stimulated PBMC.

### (Example 4)

A library of EGI cDNA tags is prepared in the same manner used in example 1 except that HpyCH4V is used instead of RsaI as a type II restriction enzyme and R1eAI is used instead of BsmFI as a second type IIS restriction enzyme. The linker X-cDNA fragment complex is prepared according to any one of processes (1), (2) or (3).
(1) The cDNAs in the sample are directly ligated to linker Xes having the structure illustrated below at the blunt end of the cDNA which was formed by cleaving with type II restriction enzyme HpyCH4V. 5'-···TGCAGCTGAGGAGTCATCCCA···-3'    (SEQ ID NO:52) 3'-···ACGTCGACTCCTCAGTAGGGT···-5'     (SEQ ID NO:53)
(2) Base "T" is ligated to the blunt end of the cDNA in the sample formed by cleaving with type II restriction enzyme HpyCH4V in the presence of dTTP in the following manner.
   5'-^{...} CA^{···}-3'
   3'-^{···}TGT^{···}-5'
   In the next step, the cohesive end above was ligated to linker X having the following structure. 5'-···TGCAGCTGAGGAGTCATCCCA···-3'          (SEQ ID NO:52) 3'-···ACGTCGACTCCTCAGTAGGG ···-5'          (SEQ ID NO:54)
(3) Base "G" was deleted from the blunt end of the cDNA in the sample formed by cleaving with type II restriction enzyme HpyCH4V in the presence of dATP, dTTP and dCTP in the following manner.
   5'-^{...}CA^{...}-3'
   3'-^{...} T^{...}-5'

In the following step, the cohesive end above was ligated to linker X having the following structure.

5'-···TGCAGCTGAGGAGTCATCCCA ···-3'          (SEQ ID NO:52)

3'-···ACGTCGACTCCTCAGTAGGGTG···-5'          (SEQ ID NO:55)

The linker X-cDNA fragments are cleaved with R1eAI by utilizing the recognition site of restriction enzyme R1eAI "5'-CCCACA-3' " formed by ligating the linker X. The mixture of cleaved products are centrifuged and the supernatant is collected. Since the cleavage site of the enzyme is at position 5'-CCCACA-3'(12/9), the collected fragments includes tags of 12 base pairs derived from the cDNA following the linker X.

Subsequently, linker Ys having the structure illustrated below are ligated. A desired library of the EGI cDNA tags are obtained by treating in the same manner as described in example 1 and digesting with the first type IIS restriction enzymes.

5'-··· CACTGTGTCGCTCCTCACTAGAC···-3' (SEQ ID NO:56)

3'-···NNNGTGACACAGCGAGGAGTGATCTG···-5'     (SEQ ID NO:57)

### (Example 5)

The gene expression may be analyzed by detecting the library of the EGI cDNA tags obtained in example 4 with a detector described below.

A DNA chip is produced by synthesizing oligo DNAs corresponding to mfID261849128, 220597775, 69402230, 232235060, 110001478 and 196314601 of the genes listed in Table 1 whose expression is activated by LPS stimulation, and spotting on a slide glass with the oligo DNAs using a conventional method.

A probe solution is prepared by labeling mRNAs derived from peripheral blood mononuear cells (PBMC) obtained by LPS stimulation in example 3 which are used as a template with a fluorescent marker, fluorescent compound Cy3-dUTP (*1) (Amersham Pharmacia), and labeling other mRNAs derived from PBMC not stimulated with LPS which are used as a template with a fluorescent marker, fluorescent compound Cy5-dUTP (*2) (Amersham Pharmacia).

The probe solutions are mixed together to make 6 X SET [0.9M NaCI, 10 µ g/ml Yeast tRNA, 0.1%SDS, 120mM Tris-HCl(pH7.8)] solution and then kept in contact with said oligo DNA chip at 45 degrees Celsius overnight to perform hybridization.

After the DNA chip is washed with a washing solution [6 × SSC, 0.1 %SDS]at 52 degrees Celsius, the fluorescent markers on the chip are scanned with a scanner to obtain the fluorescence intensity data and then the data is analyzed. The scatter plot of signal intensity of Cy3 and Cy5 at each of the spots demonstrates that the fluorescent light radiated by the probes derived from the mRNAs from the PBMC stimulated with LPS is more than twice stronger than that from the PBMC not stimulated with LPS at all of the spots.

### (Industrial Applicability)

According to the present invention, cDNAs to be tested or genes specifically expressed in cells to be tested can be accurately detected with a high reproducibility to analyze. A method of the present invention can indicate differences of gene expression between optional two kinds of cells to clarify differences in their functions and morphologies. The method is therefore applicable to analysis of huge number of aspect of biological phenomena under a physiological condition or a diseased state.

## Claims

1. A method for the preparation of cDNA tags for identifying expressed genes comprising:
providing complementary deoxyribonucleic acids (cDNAs);
cleaving the cDNAs with a type II restriction enzyme to prepare cDNA fragments;
ligating the cDNA fragments to linker Xes which have a recognition site of a first type IIS restriction enzyme and which form a recognition site of a second type IIS restriction enzyme at the site linking with the cleavage end-sites of the cDNA fragments formed by the type II restriction enzyme to prepare linker X-cDNA fragment complexes;
cleaving the linker X-cDNA fragment complexes with the second type II restriction enzyme to prepare linker X-cDNA tag complexes;
ligating linker Ys which have a recognition site of the first type IIS restriction enzyme to the cleavage end-sites of the linker X-cDNA tag complexes formed by the second type IIS restriction enzyme to prepare linker X-cDNA tag-linker Y complexes;
amplifying the linker X-cDNA fragment-linker Y complexes; and
cleaving the amplified products thus obtained with the first type IIS restriction enzymes simultaneously or in turn to prepare the cDNA tags for identifying expressed genes.

2. The method according to claim 1 further comprising the step of refining the linker X-cDNA fragment complexes.

3. The method according to claim 1 further comprising the step of processing the end-sites of the cDNA fragments in the linker X-cDNA fragment complexes to make the end-sites capable of binding to the linker Ys having a recognition site of the first type IIS restriction enzyme.

4. The method according to claim 2 further comprising the step of processing the end-sites of the cDNA fragments in the linker X-cDNA fragment complexes to make the end-sites capable of binding to the linker Ys having a recognition site of the first type IIS restriction enzyme.

5. The method according to any one of claims 1, 2, 3 or 4 further comprising the step of separating the obtained cDNA tags for identifying expressed genes.

6. The method according to claim 1 wherein the cDNAs are prepared from the mRNAs derived from cells to be examined.

7. The method according to claim 1 wherein the cDNAs are prepared from the mRNAs derived from cells to be examined using oligo-dT primers immobilized on a solid phase as an oligo-dT primer.

8. The method according to claim 7 wherein the oligo-dT primers comprise oligo-dT primers immobilized on latex beads or magnet beads.

9. The method according to claim I wherein the type II restriction enzyme has the recognition site of four base pairs.

10. The method according to claim 1 wherein the type II restriction enzyme is selected from the group consisting of AfaI, AluI, CviRI, DpnI, HpyCH4V, HpyF44III, RsaI, BfaI, Csp6I, HpyCH4IV, MaeI, MaeII, TaqA1phaI, TaqI, TthHB8I, XspI, Bsp143I, DpnII, MboI, NdeII, Sau3AI, NIaIII, AccII, Bsh1236I, BstUI, BsuRI, FnuDII, HaeIII, MvnI, AciI, BsiSI, HapII, Hin6I, HinP1I, HpaII, MspI, SciNI, CfoI, HhaI, MseI, Trull, Tru9I, TasI, Tsp509I and TspEI.

11. The method according to claim 1 wherein the first type ITS restriction enzyme is selected from the group consisting of MmeI, BpmI, BsgI, BspGI, Eco57I, GsuI, BsmFI, BcefI, FokI, BbvI, Bsp423I, Bst71I, RIeAI, Ecil, BseMII, BseRI, Hgal, LweI, SfaNI, Aprl, BspMI, HphI, MboII, MnII, BbsI, BciVI, BbvII, BpiI, BplI, BpuAI and FauI.

12. The method according to claim 1 wherein the first type IIS restriction enzyme is selected from the group consisting of Mmel, Bpml, Bsgl, BspGI, Eco57I, GsuI, BsmFI, BcefI, FekI, BbvI, Bsp423I, Bst71I, R1eAI, EciI, BseMII, BseRI and HgaI .

13. The method according to claim 1 wherein the first type IIS restriction enzyme is selected from the group consisting of MmeI, BpmI, BsgI, BspGI, Eco57I and GsuI.

14. The method according to claim I wherein the second type IIS restriction enzyme is selected from the group consisting of MmeI, BpmI, BsgI, BspGI, Eco57I, GsuI, BsmFI, BcefI, FokI, BbvI, Bsp423I, Bst71I, R1eAI, Ecil, BseMII, BseRI, HgaI, LweI, SfaNI, Apr1, BspMI, HphI, MboII, MnlI, BbsI, BciVI, BbvII, BpiI, Bp1I, BpuAI and FauI.

15. The method according to claim I wherein the second type IIS restriction enzyme is selected from the group consisting of MmeI, BpmI, BsgI, BspGI, Eco57I, GsuI, BsmFI, BcefI, FokI, BbvI, Bsp423I, Bst71I, R1eAI, EciI, BseMII, BseRI and HgaI.

16. The method according to claim 1 wherein the second type IIS restriction enzyme is selected from the group consisting of MmeI, BpmI, BsgI, BspGI, Eco57I and GsuI.

17. The method according to claim 1 wherein the type II restriction enzyme is selected from the group consisting of AfaI, RsaI, CviRI, HpyCH4V, HpyF44III, Acil, Hhal, HinP1I, Hin6I, SciNI, DpnI and CfoI and the second type IIS restriction enzyme is selected from the group consisting of MmeI, BsmFI, RIeAI, HgaI, LweI, SfaNI, Mn1I, BbsI, BbvII, BpiI, BplI, BpuAI and FauI.

18. The method according to claim 1 wherein the type II restriction enzyme is HpyCH4V, and the second type IIS restriction enzyme is R1eAI.

19. The method according to claim 1 wherein the type II restriction enzyme is AfaI, and the second type IIS restriction enzyme is BsmFI.

20. The method according to claim 1 wherein the type II restriction enzyme is RsaI, and the second type IIS restriction enzyme is BsmFI.

21. The method according to claim 1 wherein the type II restriction enzyme is HinP1I, and the second type IIS restriction enzyme is HgaI.

22. The method according to claim 1 wherein the type II restriction enzyme is AfaI, and the second type IIS restriction enzyme is MmeI.

23. The method according to claim 1 wherein the type II restriction enzyme is RsaI, and the second type IIS restriction enzyme is MmeI.

24. The method according to claim 1 wherein the length of the cDNA tags for identifying expressed genes ranges from 6 base pairs (bp) to 25 bp.

25. The method according to claim 1 wherein the length of the cDNA tags for identifying expressed genes ranges from 10 bp to 25 bp.

26. The method according to claim 1 wherein the length of the cDNA tags for identifying expressed genes ranges from 10 bp to 16 bp.

27. Linker X comprising a recognition site of the first type IIS restriction enzymes and being capable of forming a recognition site of the second type IIS restriction enzyme at the position linking to the cDNA fragment cleaved by the type II restriction enzyme.

28. The linker X according to claim 27 comprising base sequences of SEQs 12 and 13.

29. Linker X-cDNA fragment complex comprising cDNA fragment formed by cleaving with a type II restriction enzyme and linker X having a recognition site of the first type IIS restriction enzymes and being capable of forming a recognition site of the second type IIS restriction enzyme at the position linking to the cDNA fragment cleaved by the type II restriction enzyme.

30. Linker X-cDNA tag-linker Y complex wherein linker Y having a recognition site of the first type IIS restriction enzyme is ligated at the cleavage end of linker X-cDNA fragment complex of claim 29.

31. The linker X-cDNA tag-linker Y complex according to claim 29 comprising base sequence of SEQ 18 and its complementary sequence.

32. Library of cDNA tags for identifying expressed genes prepared by the method according to any one of claims 1, 2, 3 or 4.

33. A method for the analysis of gene expression wherein the library of cDNA tags according to claim 31 is contacted with a detector on which nucleic acids to be detected are immobilized.

34. The method for the analysis of gene expression according to claim 33 wherein the detector comprises DNA chip having spots on which nucleic acids to be detected are immobilized.

35. A method for the analysis of gene expression comprising the steps of concatenating cDNA tags prepared by the method according to any one of claims 1, 2, 3 or 4 each other to form concatemers and sequencing the concatemers.

36. The method according to claim 35 wherein the concatemer consists of 3 to 200 of the cDNA tags for identifying expressed genes.

37. The method according to claim 35 wherein the concatemer consists of 3 to 80 of the cDNA tags for identifying expressed genes.

38. The method according to claim 35 wherein the concatemer consists of 16 to 40 of the cDNA tags for identifying expressed genes.

39. The method for the qualitative analysis of gene expression according to claim 36 wherein the concatemers are sequenced and then each of the cDNA tags are sequenced on the basis of the sequences of the concatemers.

40. The method for the quantitative analysis of gene expression according to claim 36 wherein the concatemers are sequenced and then each of the cDNA tags are sequenced and measured in frequency of occurrences on the basis of the sequences of the concatemers.

41. A concatemer consisting of the cDNA tags prepared by the method according to any one of claims 1, 2, 3 or 4 wherein no spacer sequence exists among the cDNA tags.

42. The concatemer according to claim 41, which consists of 3 to 200 of the cDNA tags.

43. The concatemer according to claim 41, which consists of 3 to 80 of the cDNA tags.

44. The concatemer according to claim 41, which consists of 16 to 40 of the cDNA tags.

45. A concatemer consisting of the cDNA tags prepared by the method according to any one of claims 1, 2, 3 or 4 wherein spacer sequences exist among the cDNA tags.

46. The concatemer according to claim 45, which consists of 3 to 200 of the cDNA tags.

47. The concatemer according to claim 45, which consists of 3 to 80 of the cDNA tags.

48. The concatemer according to claim 45, which consists of 16 to 40 of the cDNA tags.

49. A kit for the preparation of cDNA tags for identifying expressed genes wherein the kit comprises a type II restriction enzyme, a first type IIS restriction enzyme, a second type IIS restriction enzyme, linker Xes which have a recognition site of the first type IIS restriction enzyme and which form a recognition site of the second type IIS restriction enzyme at the site linking with the cleavage end of the cDNA fragments formed by the type II restriction enzyme, and linker Ys which have a recognition site of the first type IIS restriction enzyme.

50. The kit according to claim 49 wherein the kit comprises primer Xes which hybridize the linker Xes and primer Ys which hybridize linker Ys.


Sequence Listing
SEQUENCE LISTING

<110> KUREHA CHEMICAL INDUSTRY COMPANY, LIMITED
YAMAMOTO, Mikio
YAMAMOTO, Naoki
HIROSE, Kunitaka
SAKAI, Jun

<120> METHOD FOR PREPARATION OF EXPRESSED GENE IDENTIFICATION CDNA TAG AND
METHOD FOR ANALYSIS OF GENE EXPRESSION

<130> 0701002WO1

<150> JP2001/073959

<151> 2001-03-15

<160> 57

<170> PatentIn version 2.1


<210> 1
<211> 14
<212> DNA
<213> Artificial Sequence

<220>
<223> Synthetic DNA

<220>
<221> n
<222> 7..11
<223> Optional

<400> 1
gaggagnnnn nggg 14


<210> 2
<211> 14
<212> DNA
<213> Artificial Sequence

<220>
<223> Synthetic DNA

<220>
<221> n
<222> 4..8
<223> Optional

<400> 2
cccnnnnnct cctc 14


<210> 3
<211> 15
<212> DNA
<213> Artificial Sequence

<220>
<223> Synthetic DNA

<220>
<221> n
<222> 6..15
<223> Optional

Sequence Listing

<400> 3
gggacnnnnn nnnnn 15


<210> 4
<211> 19
<212> DNA
<213> Artificial Sequence

<220>
<223> Synthetic DNA

<220>
<221> n
<222> 1..14
<223> Optional

<400> 4
nnnnnnnnnn nnnngtccc 19


<210> 5
<211> 20
<212> DNA
<213> Artificial Sequence

<220>
<223> Synthetic DNA

<220>
<221> n
<222> 6..19
<223> Optional

<400> 5
gggacnnnnn nnnnnnnnna 20


<210> 6
<211> 16
<212> DNA
<213> Artificial Sequence

<220>
<223> Synthetic DNA

<220>
<221> n
<222> 7..14
<223> Optional

<400> 6
gaggagnnnn nnnngt 16


<210> 7
<211> 15
<212> DNA
<213> Artificial Sequence

<220>
<223> Synthetic DNA

<220>
<221> n
<222> 2..9
<223> Optional

Sequence Listing

<400> 7
cnnnnnnnnc tcctc 15


<210> 8
<211> 16
<212> DNA
<213> Artificial Sequence

<220>
<223> Synthetic DNA

<220>
<221> n
<222> 7..16
<223> Optional

<400> 8
gaggagnnnn nnnnnn 16


<210> 9
<211> 14
<212> DNA
<213> Artificial Sequence

<220>
<223> Synthetic DNA

<220>
<221> n
<222> 1..8
<223> Optional

<400> 9
nnnnnnnnct cctc 14


<210> 10
<211> 16
<212> DNA
<213> Artificial Sequence

<220>
<223> Synthetic DNA

<220>
<221> n
<222> 1..14
<223> Optional

<400> 10
nnnnnnnnnn nnnnac 16


<210> 11
<211> 16
<212> DNA
<213> Artificial Sequence

<220>
<223> Synthetic DNA

<220>
<221> n
<222> 1..14
<223> Optional

Sequence Listing

<400> 11
nnnnnnnnnn nnnngt 16


<210> 12
<211> 21
<212> DNA
<213> Artificial Sequence

<220>
<223> Synthetic DNA

<400> 12
tgcagctgag gagtccatgg g 21


<210> 13
<211> 21
<212> DNA
<213> Artificial Sequence

<220>
<223> Synthetic DNA

<400> 13
cccatggact cctcagctgc a 21


<210> 14
<211> 20
<212> DNA
<213> Artificial Sequence

<220>
<223> Synthetic DNA

<400> 14
ccatggactc ctcagctgca    20


<210> 15
<211> 22
<212> DNA
<213> Artificial Sequence

<220>
<223> Synthetic DNA

<400> 15
tcccatggac tcctcagctg ca 22


<210> 16
<211> 22
<212> DNA
<213> Artificial Sequence

<220>
<223> Synthetic DNA

<400> 16
catgtgtcgc tcctcactag ac 22


<210> 17
<211> 23
<212> DNA

Sequence Listing
<213> Artificial Sequence

<220>
<223> Synthetic DNA

<400> 17
gtctagtgag gagcgacaca tgt 23


<210> 18
<211> 60
<212> DNA
<213> Artificial Sequence

<220>
<223> Synthetic DNA

<220>
<221> n
<222> 24..37
<223> Optional

<400> 18
tgcagctgag gagtccatgg gacnnnnnnn nnnnnnnaca tgtgtcgctc ctcactagac 60


<210> 19
<211> 14
<212> DNA
<213> Artificial Sequence

<220>
<223> Synthetic DNA

<220>
<221> n
<222> 1..14
<223> Optional

<400> 19
nnnnnnnnnn nnnn 14


<210> 20
<211> 98
<212> DNA
<213> Artificial Sequence

<220>
<223> Synthetic DNA

<220>
<221> n
<222> 3..16
<223> Optional

<220>
<221> n
<222> 19..32
<223> Optional

<220>
<221> n
<222> 35..48
<223> Optional

<220>
<221> n

Sequence Listing
<222> 51..64
<223> Optional

<220>
<221> n
<222> 67..80
<223> Optional

<220>
<221> n
<222> 83..96
<223> Optional

<400> 20
acnnnnnnnn nnnnnnacnn nnnnnnnnnn nnacnnnnnn nnnnnnnnac nnnnnnnnnn 60
nnnnacnnnn nnnnnnnnnn acnnnnnnnn nnnnnnac 98


<210> 21
<211> 98
<212> DNA
<213> Artificial Sequence

<220>
<223> Synthetic DNA

<220>
<221> n
<222> 3..16
<223> Optional

<220>
<221> n
<222> 19..32
<223> Optional

<220>
<221> n
<222> 35..48
<223> Optional

<220>
<221> n
<222> 51..64
<223> Optional

<220>
<221> n
<222> 67..80
<223> Optional

<220>
<221> n
<222> 83..96
<223> Optional

<400> 21
gtnnnnnnnn nnnnnngtnn nnnnnnnnnn nngtnnnnnn nnnnnnnngt nnnnnnnnnn 60
nnnngtnnnn nnnnnnnnnn gtnnnnnnnn nnnnnngt 98


<210> 22
<211> 14
<212> DNA
<213> Homo sapiens

<400> 22
agggtccttt tgca 14

Sequence Listing

<210> 23
<211> 14
<212> DNA
<213> Homo sapiens

<400> 23
ttgcgtgaaa agct 14


<210> 24
<211> 14
<212> DNA
<213> Homo sapiens

<400> 24
cccactttct gctg 14


<210> 25
<211> 14
<212> DNA
<213> Homo sapiens

<400> 25
tcagcgaatg aatg 14


<210> 26
<211> 14
<212> DNA
<213> Homo sapiens

<400> 26
caagagtttg ctcc 14


<210> 27
<211> 14
<212> DNA
<213> Homo sapiens

<400> 27
tctcctggaa atat 14


<210> 28
<211> 14
<212> DNA
<213> Homo sapiens

<400> 28
cggatgcttc cacc 14


<210> 29
<211> 14
<212> DNA
<213> Homo sapiens

<400> 29
tgtaattgag catc 14


<210> 30
<211> 14
<212> DNA

Sequence Listing
<213> Homo sapiens

<400> 30
gtgtatgacc tgga 14


<210> 31
<211> 14
<212> DNA
<213> Homo sapiens

<400> 31
cctccccggc ctgg 14


<210> 32
<211> 14
<212> DNA
<213> Homo sapiens

<400> 32
ctccctcact tctc 14


<210> 33
<211> 14
<212> DNA
<213> Homo sapiens

<400> 33
ctgtgaacca agtg 14


<210> 34
<211> 14
<212> DNA
<213> Homo sapiens

<400> 34
cccggaacgc actg 14


<210> 35
<211> 14
<212> DNA
<213> Homo sapiens

<400> 35
caatacgagt tccc 14


<210> 36
<211> 14
<212> DNA
<213> Homo sapiens

<400> 36
tctgcttgcg gagg 14


<210> 37
<211> 14
<212> DNA
<213> Homo sapiens

<400> 37
ccccttctgg gcat 14

Sequence Listing

<210> 38
<211> 14
<212> DNA
<213> Homo sapiens

<400> 38
caggcagtgc gggc 14


<210> 39
<211> 14
<212> DNA
<213> Homo sapiens

<400> 39
tacgttgtag ctca 14


<210> 40
<211> 14
<212> DNA
<213> Homo sapiens

<400> 40
caacagcagc catg 14


<210> 41
<211> 14
<212> DNA
<213> Homo sapiens

<400> 41
tgagacctag agtc 14


<210> 42
<211> 14
<212> DNA
<213> Artificial Sequence

<220>
<223> Synthetic DNA

<400> 42
aaaaaaaaaa aaaa 14


<210> 43
<211> 14
<212> DNA
<213> Artificial Sequence

<220>
<223> Synthetic DNA

<400> 43
aaaaaaaaaa aaac 14


<210> 44
<211> 14
<212> DNA
<213> Artificial Sequence

<220>
<223> Synthetic DNA

Sequence Listing

<400> 44
aaaaaaaaaa aaag 14


<210> 45
<211> 14
<212> DNA
<213> Artificial Sequence

<220>
<223> Synthetic DNA

<400> 45
aaaaaaaaaa aaat 14


<210> 46
<211> 14
<212> DNA
<213> Artificial Sequence

<220>
<223> Synthetic DNA

<400> 46
aaaaaaaaaa aaca 14


<210> 47
<211> 14
<212> DNA
<213> Artificial Sequence

<220>
<223> Synthetic DNA

<400> 47
tttttttttt ttgt 14


<210> 48
<211> 14
<212> DNA
<213> Artificial Sequence

<220>
<223> Synthetic DNA

<400> 48
tttttttttt ttta 14


<210> 49
<211> 14
<212> DNA
<213> Artificial Sequence

<220>
<223> Synthetic DNA

<400> 49
tttttttttt tttc 14


<210> 50
<211> 14
<212> DNA

Sequence Listing
<213> Artificial Sequence

<220>
<223> Synthetic DNA

<400> 50
tttttttttt tttg 14


<210> 51
<211> 14
<212> DNA
<213> Artificial Sequence

<220>
<223> Synthetic DNA

<400> 51
tttttttttt tttt 14


<210> 52
<211> 21
<212> DNA
<213> Artificial Sequence

<220>
<223> Synthetic DNA

<400> 52
tgcagctgag gagtcatccc a 21


<210> 53
<211> 21
<212> DNA
<213> Artificial Sequence

<220>
<223> Synthetic DNA

<400> 53
tgggatgact cctcagctgc a 21


<210> 54
<211> 20
<212> DNA
<213> Artificial Sequence

<220>
<223> Synthetic DNA

<400> 54
gggatgactc ctcagctgca 20


<210> 55
<211> 22
<212> DNA
<213> Artificial sequence

<220>
<223> Synthetic DNA

<400> 55
gtgggatgac tcctcagctg ca 22

Sequence Listing

<210> 56
<211> 23
<212> DNA
<213> Artificial Sequence

<220>
<223> Synthetic DNA

<400> 56
cactgtgtcg ctcctcacta gac 23


<210> 57
<211> 26
<212> DNA
<213> Artificial Sequence

<220>
<221> Optional
<222> 24..26
<223> n

<400> 57
gtctagtgag gagcgacaca gtgnnn 26